# EUROPEAN PATENT APPLICATION

(11) **EP 4 179 999 A1**
(43) Date of publication of application: **17.05.2023**
(21) Application number: 21836861.1
(22) Date of filing: 07.07.2021
(51) Int. Cl.: A61B 46/20, A61F 5/445

(54) **ORGAN PROTECTING MEMBER**

(30) Priority: 07.07.2020 JP 2020116993; 29.06.2021 JP 2021107211
(71) Applicant: National Cancer Center, Tokyo 104-0045 (JP); Sumitomo Bakelite Co., Ltd., Shinagawa-ku Tokyo 140-0002 (JP)
(72) Inventor: ITO, Masaaki, Tokyo 104-0045 (JP); HASEGAWA, Hiro, Tokyo 104-0045 (JP); SUZUKI, Zenetsu, Akita-shi, Akita 011-8510 (JP); KAWAMATA, Akira, Akita-shi, Akita 011-8510 (JP)
(74) Representative: Ter Meer Steinmeister & Partner
(86) International application number: PCT/JP2021/025597
(87) International publication number: WO 2022/009920

(57) **Abstract**

This organ protecting member (1) is used when an opening portion (36a) is formed in an abdominal wall (30) and an intestinal tract (32) is exposed to a body surface (33) from the opening portion (36a) to temporarily construct an artificial anus (34). The organ protecting member (1) includes a movement restricting part (support string (12)) configured to restrict movement of a portion of the lifted intestinal tract (32) on which the artificial anus (34) is constructed toward an abdominal cavity (35); and a cover (11) disposed between the intestinal tract (32) and an inner wall surface (36b) of the opening portion (36a) of the abdominal wall (30).

## Description

### Technical Field

The present invention relates to an organ protecting member that protects an intestinal tract for constructing an artificial anus.

Priority is claimed on Japanese Patent Application No. 2020-116993, filed on July 7, 2020 and Japanese Patent Application No. 2021-107211, filed on June 29, 2021, the contents of which are incorporated herein by reference.

### Background Art

In rectal surgery, an opening portion may be provided in the abdomen to lift the intestinal tract and construct an artificial anus (hereinafter, also referred to as a stoma). Specifically, a lesion portion of the intestinal tract on an anal side is excised, and the intestines separated by this excision are sutured together. An artificial anus is provided on an upstream side (oral side) of the sutured site so as to prevent feces and digestive juices from inflowing and causing a suture failure.

This artificial anus has two openings in the intestinal tract on the oral side and in the intestinal tract on the anal side and is thus called a loop artificial anus. A subject connects a pouch for storing waste (feces) to a periphery of the artificial anus and excretes the waste from the pouch.

For example, PTL 1 discloses a surgical anastomosis device that is attached around an artificial anus, has a ring-shaped protrusion, and is connected to a pouch having a ring-shaped groove, thereby preventing waste from leaking out of the pouch.

Conversely, PTL 2 discloses an excreta storage device that is attached around an artificial anus, has a ring-shaped groove, and is connected to a pouch having a ring-shaped protrusion, thereby preventing waste from leaking out of the pouch.

However, as one of the most serious postoperative complications in rectal surgery, there is a suture failure. A suture failure is a situation in which the anastomosed intestinal tract fails without being fused together, and contents of the intestinal tract such as digestive juices and feces leak into the abdominal cavity. In rectal surgery, a suture failure rate is as high as about 10%. In order to avoid this suture failure, a procedure is known in which a temporary artificial anus (loop stoma) is constructed at the same time as excision of a lesion portion.

In rectal surgery, surgery may be performed in two stages, in which an artificial anus is used for about 3 months after a construction operation, and an operation of closing the artificial anus is performed within 1 year after about 3 months. Therefore, a subject requires a total of two hospitalizations, one for excision of a lesion portion and construction of a temporary artificial anus, and the other for closure of the artificial anus.

In recent years, papers and the like have reported the effectiveness of closing an artificial anus as early as within 14 days after an initial operation for subjects who do not undergo a suture failure. As described above, when a period until the closure of the artificial anus is shortened, it is possible to complete the operation for constructing the artificial anus and the closing operation in one hospitalization.

In addition, when a period during which an artificial anus is provided is shortened, especially when a subject can be kept under the supervision of a doctor for a single hospitalization, stoma-related complications such as skin disorders and wound infections in a periphery of the artificial anus, intestinal obstruction, hernia, or falling and necrosis of the artificial anus can be reduced. Therefore, an improvement in quality of life (QOL) of the subject is expected.

Early artificial anus closure also contributes to a reduction in economic burden of stoma appliances and stoma supplies.

### Citation List

### Patent Literature

[PTL 1] Japanese Unexamined Patent Application, First Publication No. 2002-345872
[PTL 2] Japanese Unexamined Patent Application, First Publication No. 2006-296634

### Summary of Invention

### Technical Problem

However, when the artificial anus is closed early after the initial operation, a tissue reaction may occur between the intestinal tract that is lifted toward the outside of the body for the construction of the artificial anus and the abdominal wall (an inner wall surface of an opening portion in the abdominal wall), and a separating operation or the like is necessary. Accordingly, the difficulty of the surgery is high at times.

Therefore, there is a clinical need for early closure of a temporary artificial anus during rectal surgery in a more stable state.

The present invention has been made in view of the problems described above, and provides an organ protecting member capable of suppressing direct contact of the intestinal tract adjacent to the abdominal wall with the abdominal wall for construction of an artificial anus, and of satisfying a clinical need.

### Solution to Problem

An organ protecting member of the present invention is an organ protecting member used when an opening portion is formed in an abdominal wall and an intestinal tract is exposed to a body surface from the opening portion to temporarily construct an artificial anus, and includes: a movement restricting part configured to restrict movement of a portion of the intestinal tract on which the artificial anus is constructed toward an abdominal cavity; and a cover disposed between the intestinal tract and an inner wall surface of the opening portion.

### Advantageous Effects of Invention

According to the organ protecting member of the present invention, it is possible to suppress direct contact between the intestinal tract that is adjacent to the opening portion of the abdominal wall for the construction of the artificial anus and the inner wall surface of the opening portion of the abdominal wall. Accordingly, a tissue reaction between the intestinal tract and the abdominal wall, which is a factor that increases a difficulty of surgery, can be suppressed, and a separating operation is not necessary, which can satisfy clinical needs.

### Brief Description of Drawings

FIG. 1 is a schematic view showing an artificial anus constructed in a subject and a pouch attached to the artificial anus.
FIG. 2 is a schematic cross-sectional view showing a state in which an organ protecting member according to a first embodiment is attached to a periphery of the artificial anus and the pouch is attached to the organ protecting member.
FIG. 3A is a plan view of the organ protecting member.
FIG. 3B is a front view of the organ protecting member.
FIG. 4A is a plan view of a locking member.
FIG. 4B is a front view of the locking member.
FIG. 5 is an explanatory view showing a state in which a portion of an intestinal tract is lifted to an outside of a body.
FIG. 6 is an explanatory view showing a state in which the organ protecting member is attached to the intestinal tract;
FIG. 7 is an explanatory view showing an operation of passing a support string through the intestinal tract and a cover under the lifted intestinal tract.
FIG. 8 is an explanatory view showing a state in which the intestinal tract is lowered to a position where the intestinal tract is supported by the support string, and an external cover part is sutured to a top portion of the lifted intestinal tract.
FIG. 9 is an explanatory view showing a state in which the lifted intestinal tract and the organ protecting member are moved to a position where a body surface plate lower portion of the locking member abuts a body surface, and the top portion of the intestinal tract is incised.
FIG. 10 is an explanatory view showing a state in which an incision portion formed at the top portion of the intestinal tract is folded radially outward.
FIG. 11 shows an operation of closing the artificial anus, shows a state in which an internal cover part is taken out of the body, and is an explanatory view showing an anastomosed portion between an oral side intestinal tract and an anal side intestinal tract and sutured and cut portions.
FIG. 12 is a perspective view showing an organ protecting member according to a second embodiment.
FIG. 13 is an explanatory view showing an operation of passing a support string according to the second embodiment through the intestinal tract and a cover.
FIG. 14 is an explanatory view showing a state in which the intestinal tract is fixed to the body surface.
FIG. 15 is an explanatory view describing a reinforcing seal according to a first modification example.
FIG. 16 is an explanatory view describing a seat valve according to a second modification example.
FIG. 17A is a perspective view describing a suture reinforcing part of the external cover part.
FIG. 17B is a longitudinal cross-sectional view describing the suture reinforcing part of the external cover part shown in FIG. 17A.

### Description of Embodiments

Hereinafter, embodiments of the present invention will be described with reference to the drawings. The embodiments described below are merely examples for facilitating understanding of the present invention, and do not limit the present invention. That is, shapes, dimensions, dispositions, and the like of members described below can be changed and improved without departing from the gist of the present invention, and the present invention naturally includes equivalents thereof.

It should be noted that various constituent elements of the present invention do not have to exist independently of each other. That is, it is permitted that a plurality of constituent elements are configured as a single constituent element, a single constituent element is divided into a plurality of constituent elements, a constituent element is a part of another constituent element, a part of a constituent element is a part of another constituent element, and the like.

In addition, in all the drawings, the same constituent elements are denoted by the same reference numerals, and overlapping descriptions are omitted as appropriate.

### <First Embodiment>

### <Overview>

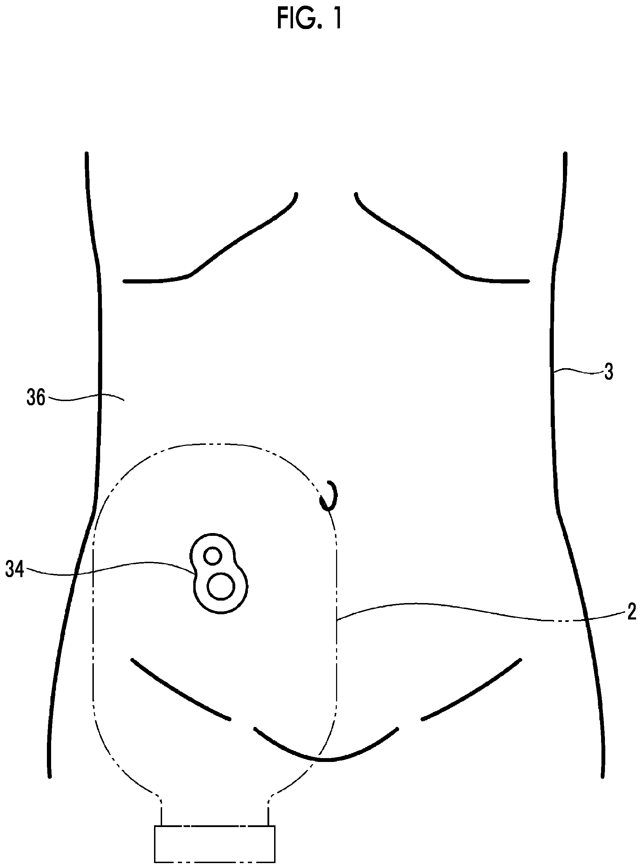

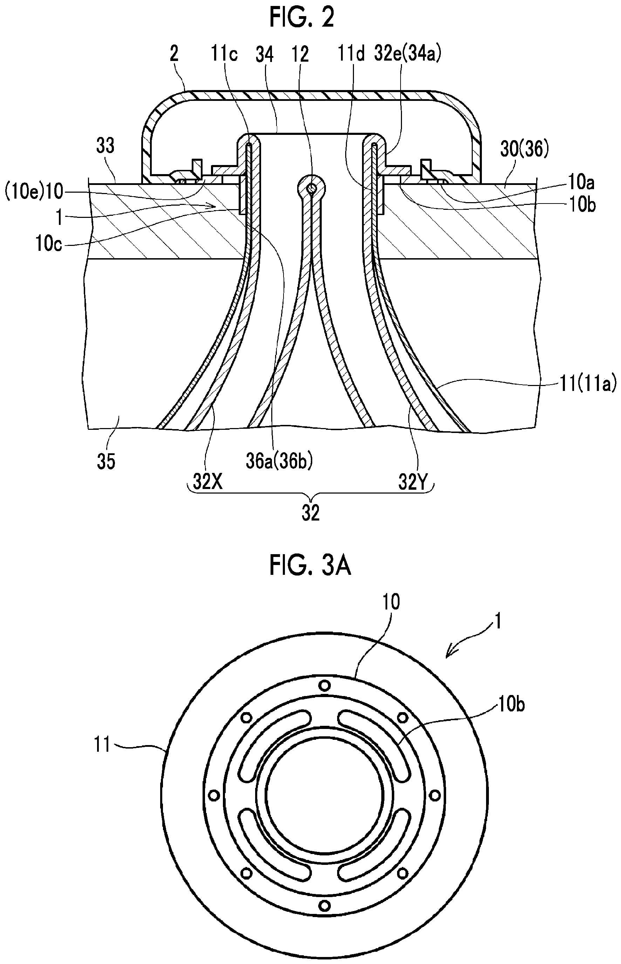

First, an overview of an organ protecting member 1 according to a first embodiment will be described mainly with reference to FIGS. 1 and 2.

FIG. 1 is a schematic view showing an artificial anus 34 constructed in a subject 3 and a pouch 2 attached to the artificial anus 34. FIG. 1 shows a state in which a general pouch 2 is used, and the organ protecting member 1 is omitted in FIG. 1.

FIG. 2 is a schematic cross-sectional view showing a state in which the organ protecting member 1 according to the present embodiment is attached to a periphery of the artificial anus 34 and the pouch 2 is attached to the organ protecting member 1 (locking member 10 described later).

As shown in FIG. 2, the organ protecting member 1 is used when an opening portion 36a is formed in an abdominal wall 30, and an intestinal tract 32 (of the small intestine (ileum)) is exposed to a body surface 33 from the opening portion 36a to temporarily construct the artificial anus 34 (loop stoma).

The organ protecting member 1 includes a movement restricting part (the locking member 10 and a support string 12 in the present embodiment) that restricts movement of a portion of the lifted intestinal tract 32 on which the artificial anus 34 is constructed toward an abdominal cavity 35, and a cover 11 disposed between the intestinal tract 32 and an inner wall surface 36b of the opening portion 36a of the abdominal wall 30.

The artificial anus 34 is not a permanent artificial anus, but is a temporary artificial anus that is constructed on the intestinal tract 32 and is returned to an inside of a body after excising and suturing, for example, rectal/colon cancers (lesion portions) and confirming that there is no suture failure.

The "movement restricting part" may be any structure that can restrict the movement of the portion of the lifted intestinal tract 32 on which the artificial anus 34 is constructed toward the abdominal cavity 35, and includes at least one of the support string 12 and the locking member 10.

The "movement restricting part" in the present embodiment includes the support string 12 and the locking member 10. In addition, the "movement restricting part" further includes an external cover part 11c that restricts movement of the intestinal tract 32 in a case where the portion of the intestinal tract 32 is sutured to the external cover part 11c of the cover 11.

The support string 12 supports the intestinal tract 32 from below to restrict the movement of the intestinal tract 32.

The locking member 10 fixes the intestinal tract 32 to the external cover part 11c, and the locking member 10 joined to the external cover part 11c is sutured and fixed to the body surface 33, thereby restricting the movement of the intestinal tract 32.

The intestinal tract 32 is sutured and fixed to the external cover part 11c, and the external cover part 11c is sutured and fixed to the body surface 33, thereby restricting the movement.

According to the above configuration, the organ protecting member 1 can suppress direct contact between the intestinal tract 32 that is temporarily adjacent to the opening portion 36a of the abdominal wall 30 for the construction of the artificial anus 34 and the inner wall surface 36b of the opening portion 36a of the abdominal wall 30 using the cover 11 while stabilizing a position of the intestinal tract 32 using the movement restricting part.

### <Configuration of Each Part>

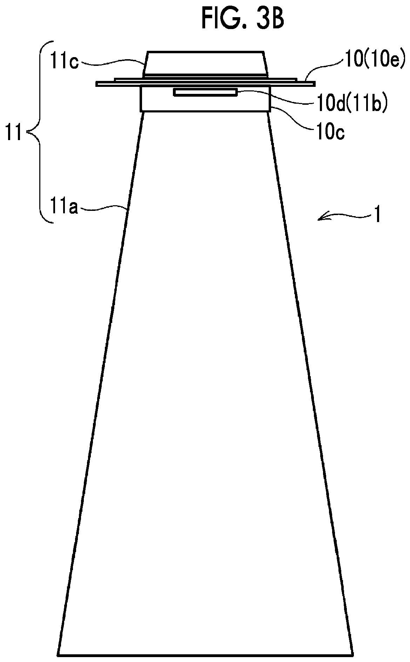

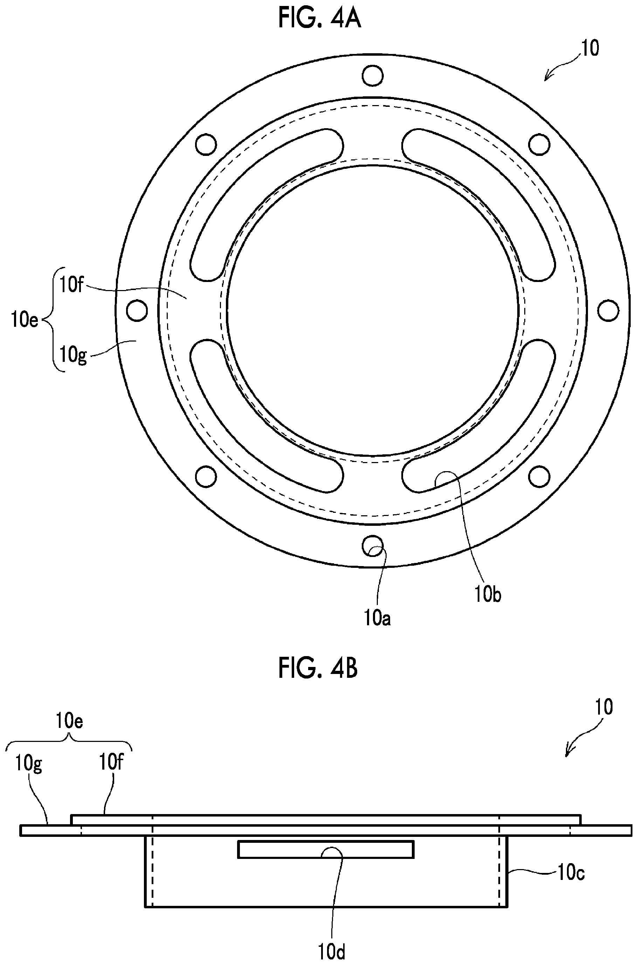

Next, a configuration of each part of the organ protecting member 1 will be specifically described mainly with reference to FIGS. 3A to 4B in addition to FIGS. 1 and 2. FIGS. 3A and 3B are views showing the organ protecting member 1, in which FIG. 3A is a plan view and FIG. 3B is a front view. FIGS. 4A and 4B are views showing the locking member 10, in which FIG. 4A is a plan view and FIG. 4B is a front view.

### [Cover]

The cover 11 according to the present embodiment prevents the lifted intestinal tract 32 from coming into direct contact with the inner wall surface 36b of the opening portion 36a of the abdominal wall 30, and serves as a base for the construction (folding of the intestinal tract 32) of the artificial anus 34.

The cover 11 is formed of silicone, polyurethane, flexible PVC, or the like, and is softer than the locking member 10 described later.

As shown in FIG. 2, the cover 11 includes a straight cylindrical part 11d connected to the locking member 10, and an internal cover part 11a that is disposed on an internal side with respect to a portion (straight cylindrical part 11d) connected to the locking member 10, and an external cover part 11c disposed on an external side, which are continuously formed. The straight cylindrical part 11d is provided between the internal cover part 11a and the external cover part 11c.

### [Internal Cover part]

The internal cover part 11a is configured to prevent a portion of the intestinal tract 32 remaining in the abdominal cavity 35 from coming into direct contact with the inner wall surface 36b of the opening portion 36a of the abdominal wall 30.

The internal cover part 11a (cover 11) according to the present embodiment is formed in a shape that flares outward from the external side toward the internal side (abdominal cavity 35).

Specifically, the term "external side of the cover 11" is on an upper side in FIG. 3B and on the locking member 10 side, whereas the "internal side of the cover 11" is on a lower side in FIG. 3B and on a terminal side.

The "shape that flares outward from the external side toward the internal side" refers to a shape that expands in diameter in a longitudinal direction from, of a pair of parallel open end walls of the internal cover part 11a, the end wall on the straight cylindrical part 11d toward the end wall on the other side.

As will be described later, the intestinal tract 32 of which a part has been lifted to an outside of the body is disposed in the body so as to spread to an oral side and an anal side as shown in FIG. 2.

As described above, since the cover 11 (internal cover part 11a) is formed in a flared shape, the cover 11 is suitable to cover a portion of the intestinal tract 32 on the inside of the body so as to separate the portion of the intestinal tract 32 from around the opening portion 36a of the abdominal wall 30.

In addition, in the cover 11 (internal cover part 11a), pleats (folds) extending in the longitudinal direction may be formed.

By forming the pleats as described above, flexibility of the cover 11 (internal cover part 11a) in a radial direction (a direction perpendicular to an axial direction) can be increased. Therefore, it becomes easy to perform an operation of covering the lifted intestinal tract 32 with the cover 11 or of putting the internal cover part 1 1a in a state of covering a portion of the intestinal tract 32 into the body together with the portion of the intestinal tract 32.

### [Straight cylindrical part]

The straight cylindrical part 11d is a part that is joined to an inner peripheral surface of the locking member 10 (extending part 10c) with an adhesive or the like. The straight cylindrical part 11d is formed shorter than the internal cover part 11a in an up-down direction (longitudinal direction) in FIG. 3B. The straight cylindrical part 11d may have a shape that decreases in diameter in the longitudinal direction from, of a pair of parallel open end walls of the straight cylindrical part 11d, the end wall on the internal cover part 11a side toward the end wall on the external cover part 11c side, but preferably has a shape having a constant diameter (that does not change in the longitudinal direction).

### [External cover part]

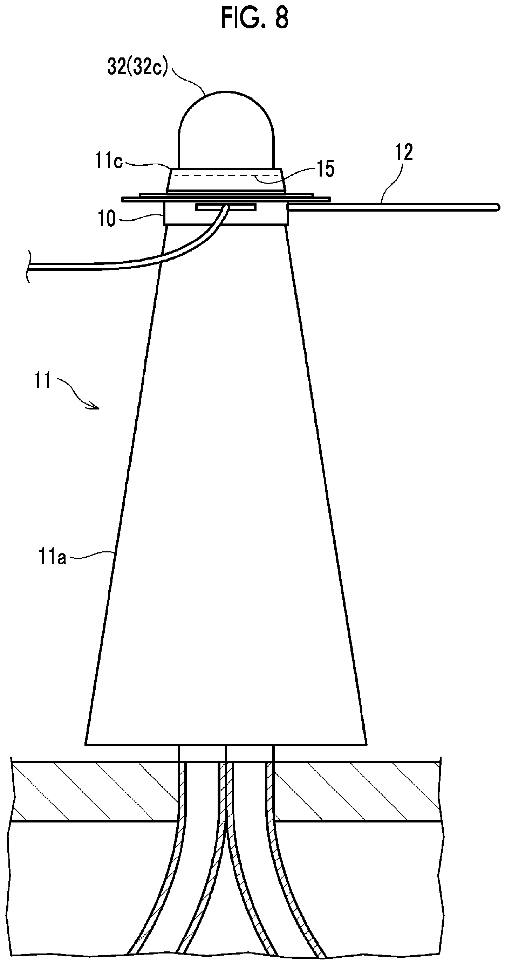

The external cover part 11c is a part that is sutured and fixed to a portion of the intestinal tract 32 pulled out of the body on which the artificial anus 34 is constructed, with a suture thread 15 (see FIG. 8).

In the present embodiment, an example in which a position at which the external cover part 11c is sutured and fixed with the suture thread 15 is an upper side of the external cover part 11c.

With such a configuration, as will be described later, when the intestinal tract 32 is folded radially outward so as to cover an upper end of the external cover part 11c, the folded portion of the intestinal tract 32 is less likely to be separated from the external cover part 11c. Therefore, the intestinal tract 32 can be easily folded along the upper end of the external cover part 11c, which is preferable.

Here, the present invention is not limited to such a configuration, and the position of the external cover part 11c to be sutured and fixed with the suture thread 15 is any position as long as the external cover part 11c can be fixed to the intestinal tract 32, and may be on a lower side of the external cover part 11c, or a plurality of points.

For example, when the lower side of the external cover part 11c is sutured and fixed, the movement of the intestinal tract 32 can be restricted from a side closer to the inside of the body to an outside in the radial direction, so that closure of an opening of the artificial anus 34 can be suppressed.

The external cover part 11c is disposed on the external side (on the upper side in FIG. 3B) from the straight cylindrical part 11d (or the locking member 10 described later).

As shown in FIG. 2, the external cover part 11c has a length and flexibility such that a top portion opening 32d of the intestinal tract 32, which will be described later, can be kept in a state of spreading out in a state in which the portion of the lifted intestinal tract 32 is folded radially outward.

That is, communication of the artificial anus 34 between the inside of the body and the outside of the body can be suitably maintained by the external cover part 11c.

The external cover part 11c is formed in a tapered shape that tapers upward (in the longitudinal direction from, of a pair of parallel open end walls of the external cover part 11c, the end wall on the straight cylindrical part 11d side toward the end wall on the other side). By forming the external cover part 11c as described above, the artificial anus 34 constructed along the external cover part 11c can be tapered upward, so that a size thereof can be made compact.

In clinical practice, a length of the cover 11 can be adjusted by excising a portion of the cover 11 in a case where a vertical length thereof is too long with respect to the lifted intestinal tract 32.

In particular, by excising the external cover part 11c and adjusting a length thereof, the intestinal tract 32 can be easily reversed (folded back).

As described above, when the external cover part 11c is sutured and fixed to the portion of the intestinal tract 32, it becomes difficult for the folded portion of the intestinal tract 32 to separate from the external cover part 11c. Therefore, the intestinal tract 32 can be easily reversed along the upper end of the external cover part 11c, which is preferable. However, the present invention is not limited to such a configuration.

For example, the external cover part 11 c is not sutured and fixed to the portion of the intestinal tract 32, but the portion of the intestinal tract 32 may be sutured and fixed to the locking member 10. Furthermore, the cover 11 does not necessarily have to include the external cover part 11c.

### [Locking Member]

The movement restricting part includes the locking member 10 that is connected to the cover 11 and comes into surface contact with and locks the body surface 33.

The locking member 10 is sutured and fixed to a skin of an abdomen 36 in a state in which the intestinal tract 32 is sutured and fixed. The pouch 2 is connected to the locking member 10. The locking member 10 is formed of silicone, polyurethane, flexible PVC, or the like.

According to the above configuration, the locking member 10 connected to the cover 11 comes into surface contact with and locks the body surface 33, so that a load applied to the body surface 33 can be distributed when a portion of the intestinal tract 32 tries to return to the abdominal cavity 35. More specifically, the load is applied from the locking member 10 to the body surface 33 via the cover 11 sutured and fixed to the intestinal tract 32 with the suture thread 15, so that the load applied to the body surface 33 and the intestinal tract 32 can be distributed.

The locking member 10 includes a body surface plate 10e and the cylindrical extending part 10c extending downward from the body surface plate 10e.

The locking member 10 is formed in a plate piece shape, preferably in a hollow disc shape. In the locking member 10, a through-hole 10a through which a suture thread (not shown) for suturing and fixing the locking member 10 to the body surface can be passed is formed in a thickness direction (plate thickness direction of the body surface plate 10e). More specifically, the body surface plate 10e of the locking member 10 is formed in a plate piece shape, preferably in a hollow disc shape.

Furthermore, as shown in FIGS. 4A and 4B, the body surface plate 10e includes an upper body surface plate upper portion 10f located on an inside the radial direction and a lower body surface plate lower portion 10g that is formed continuously with the body surface plate upper portion 10f and is formed to widen toward a radially outward side. As shown in FIG. 4A, a concentric opening portion is formed by the body surface plate upper portion 10f and the body surface plate lower portion 10g.

The through-hole 10a according to the present embodiment is formed in the body surface plate lower portion 10g and is a round hole formed in the thickness direction. For example, eight through-hole 10a are evenly provided in a circumferential direction.

The locking member 10 can be reliably fixed to the body surface 33 by passing a suture thread (not shown) through the through-holes 10a and suturing and fixing the locking member 10 to the abdominal wall 30 (body surface 33). In particular, since the locking member 10 and the body surface 33 are sutured and fixed to each other, a pulling force applied to the intestinal tract 32 and the body surface 33 can be distributed compared to a case where the intestinal tract 32 is directly sutured and fixed to the body surface 33, which is preferable.

In the locking member 10, a passage hole 10b through which a suture thread (not shown) for suturing and fixing a portion of the intestinal tract 32 forming the periphery of the artificial anus 34 to the body surface 33 can be passed is formed in the thickness direction.

As shown in FIG. 4A, the passage hole 10b according to the present embodiment is formed in the body surface plate upper portion 10f and is an arc-shaped elongated hole formed in the thickness direction. For example, four passage holes 10b are evenly provided in the circumferential direction.

By passing the suture thread (not shown) through the passage holes 10b, a portion of the intestinal tract 32 turned inside out outside the body can be reliably sutured and fixed to the body surface 33. According to the above configuration, when suturing the intestinal tract 32 to the body surface 33, it is possible to prevent the locking member 10 from becoming an obstacle.

### [Extending part]

The locking member 10 includes the extending part 10c extending toward the inside of the body. As shown in FIGS. 4A and 4B, the extending part 10c is continuously integrated with the body surface plate upper portion 10f through the body surface plate lower portion 10g to form a concentric opening portion.

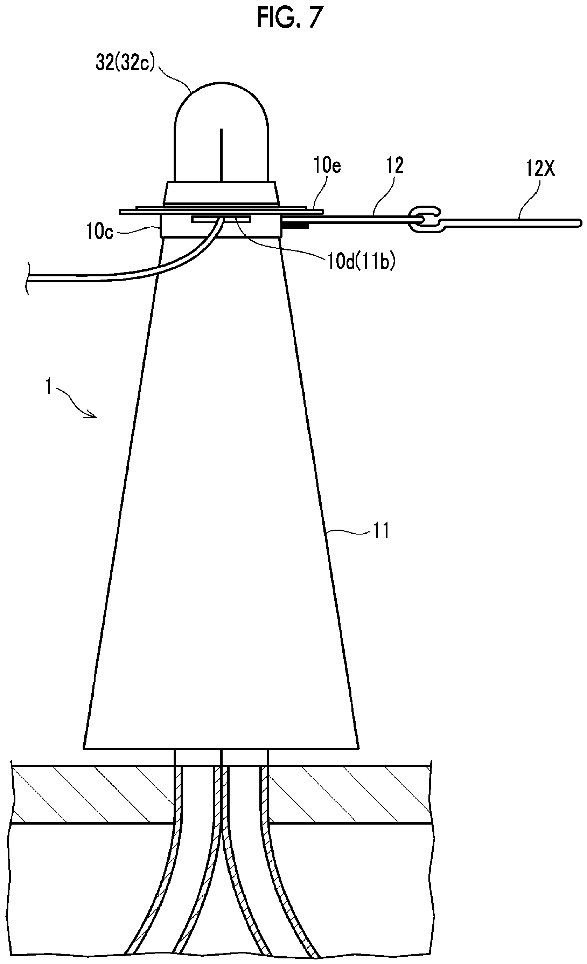

As shown in FIG. 3B, holes (slits 10d and 11b) through which the support string 12 (shown in FIGS. 2 and 7 and described later) is passed are formed in the extending part 10c and the cover 11.

The support string 12, which will be described later, is passed through the slits 10d and 11b to prevent the intestinal tract 32 from returning to the abdominal cavity 35. By passing the support string 12 through the extending part 10c of the locking member 10 and the cover 11, a position of the support string 12 can be stabilized.

The slits 10d and 11b are provided so as to overlap each other, and two of each of the slits 10d and 11b are provided at positions shifted by 180 degrees in the circumferential direction, in other words, at positions on a straight line.

In addition, the slits 10d and 11b are formed longer in a lateral (horizontal) direction than in the up-down (vertical) direction. By forming the slits 10d and 11b as described above, (lateral positions) left and right positions of the support string 12 can be easily aligned so that the support string 12 is passed under a top portion 32c of the intestinal tract 32 having an inverted U shape.

The extending part 10c is formed to extend downward in order to reduce a height of a portion exposed from the body surface 33.

### [Support String]

The movement restricting part includes the support string 12 (see FIGS. 2 and 7) that is passed under a portion of the intestinal tract 32 that is pulled out to the body surface 33 and then tries to return to the abdominal cavity 35 and supports the portion of the intestinal tract 32 from the inside of the body.

In other words, "passed under the portion of the intestinal tract 32" means that passed to support the lifted intestinal tract 32 from below.

According to the above configuration, by the support string 12 supporting the intestinal tract 32 from below, falling of the intestinal tract 32 into the body (abdominal cavity 35) can be restricted.

The support string 12 is formed of silicone, polyurethane, flexible PVC, or the like.

In addition, since the support string 12 is passed through the slit 11b of the internal cover part 11a, it is possible to prevent the cover 11 from moving downward (toward the abdominal cavity 35).

A portion of the support string 12 supporting the intestinal tract 32 from below is located in contact with a lower surface (abdominal cavity 35 side) of the body surface plate 10e (body surface plate lower portion 10g) of the locking member 10 thereunder.

The support string 12 according to the present embodiment is provided separately from the locking member 10, but the present invention is not limited to such a configuration. One end of the support string 12 may be formed to be fixed to the extending part 10c of the locking member 10.

For example, one end of the support string 12 may be fixed to the lower surface of the body surface plate 10e of the locking member 10. In addition, in a case where one end of the support string 12 is fixed to the inner peripheral surface of the locking member 10 (extending part 10c), only one of each of the slits 10d and 11b may be provided on an opposing position side at a position shifted by 180 degrees in the circumferential direction with respect to the extending part 10c to which the support string 12 is fixed.

### [Pouch]

A back side of a fixing portion of the pouch 2 is connected to the locking member 10 with an adhesive. In order to improve a leakage prevention function, for example, the pouch 2 may be configured to be fitted into the body surface plate 10e which is a portion of the locking member 10 exposed on the body surface 33. For example, the pouch 2 may also be provided with a ring (not shown) or the like having a high hardness, and this ring or the like may be fitted into the body surface plate 10e.

### <Method of Constructing Artificial Anus>

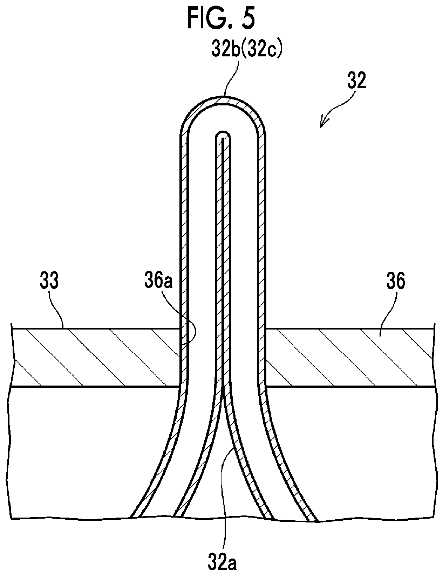

Next, a method of constructing an artificial anus according to the present embodiment will be described mainly with reference to FIGS. 5 to 10 in addition to FIG. 2.

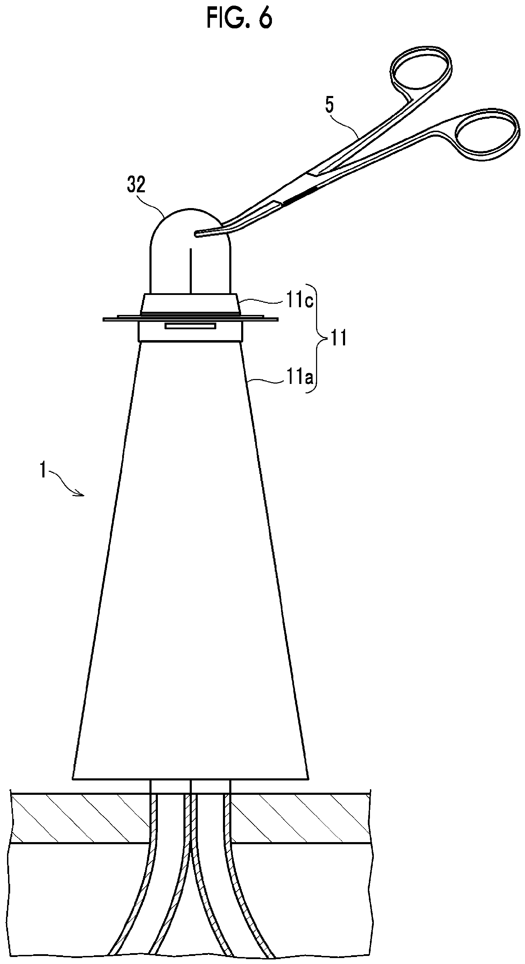

FIG. 5 is an explanatory view showing a state in which a portion of the intestinal tract 32 is lifted to the outside of the body, and FIG. 6 is an explanatory view showing a state in which the organ protecting member 1 is attached to the intestinal tract 32.

FIG. 7 is an explanatory view showing an operation of passing the support string 12 through the intestinal tract 32 and the cover 11 under the lifted intestinal tract 32. FIG. 8 is an explanatory view showing a state in which the intestinal tract 32 is lowered to a position where the intestinal tract 32 is supported by the support string 12, and the external cover part 11c is sutured to the top portion 32c of the lifted intestinal tract 32.

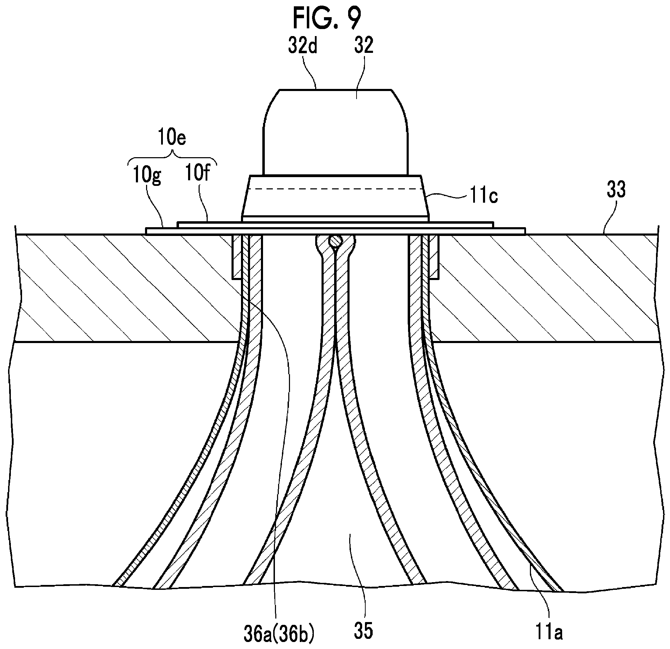

FIG. 9 is an explanatory view showing a state in which the lifted intestinal tract 32 and the organ protecting member 1 are moved downward to a position where the body surface plate lower portion 10g of the locking member 10 abuts the body surface 33, and the top portion 32c of the intestinal tract 32 is incised.

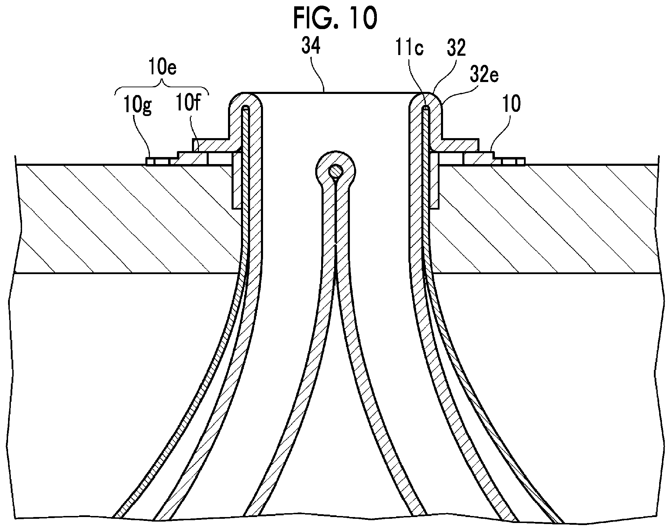

FIG. 10 is an explanatory view showing a state in which an open end portion 32e of an incision portion formed at the top portion 32c of the intestinal tract 32 is folded radially outward.

In addition, FIG. 5 is a cross-sectional view, and FIGS. 6 to 9 and FIG. 11, which will be described later, show outer shapes of the organ protecting member 1 and the intestinal tract 32 on the outside of the body in a front view and show the organ protecting member 1 and the intestinal tract 32 on the inside of the body in a cross-sectional view.

The method of constructing an artificial anus according to the present embodiment is a method of constructing the artificial anus 34 by providing the opening portion 36a in the abdominal wall 30 and lifting the intestinal tract 32 from the opening portion 36a.

In the method of constructing an artificial anus according to the present embodiment, the organ protecting member 1 is prepared after a surgical operation, for example, for rectal cancer, and an incision process of forming the opening portion 36a by making a small incision in the abdomen 36 (abdominal wall 30), which is to be a portion on which the artificial anus 34 is constructed. Furthermore, a construction process of constructing a loop stoma (artificial anus 34) by disposing at least a portion of the cover 11 (opening portion 36a) between the intestinal tract 32 and the inner wall surface 36b of the opening portion 36a is included.

### <Construction Process>

Specifically, in the construction process, an operator inserts intestinal forceps 5 (see FIG. 6) into the opening portion 36a and lifts the intestinal tract 32 to the outside of the body through the opening portion 36a so as to expose the intestinal tract 32 to the body surface 33 as shown in FIG. 5.

Next, the operator inserts the intestinal tract 32 into the internal cover part 11a of the organ protecting member 1 from below, grasps the top portion 32c of the intestinal tract 32 with the intestinal forceps 5 as shown in FIG. 6, and pulls up the top portion 32c above the external cover part 11c.

In this manner, a portion of the intestinal tract 32 disposed inside the body can be covered with the internal cover part 11a.

Next, the operator passes the support string 12 through an inner peripheral side 32a (mesentery) of the top portion 32c of the intestinal tract 32 having an inverted U shape as shown in FIG. 7. Specifically, the operator linearly passes the support string 12 through the slits 10d and 11b of the locking member 10 and the cover 11 using a string threader 12X or the like.

At this time, the intestinal tract 32 is lifted to the outside of the body sufficiently to pass through the organ protecting member 1. Therefore, the operator moves the intestinal tract 32 downward with respect to the organ protecting member 1 to an appropriate position for constructing the artificial anus 34.

Then, as shown in FIG. 8, the operator sutures and fixes the external cover part 11c to an outer peripheral side 32b (serosa) of the top portion 32c having an inverted U shape in the intestinal tract 32 pulled to the outside of the body with the suture thread 15. Accordingly, the cover 11 is connected to the intestinal tract 32.

Next, the operator returns the internal cover part 11a covering the intestinal tract 32 into the abdominal cavity 35.

At this time, the operator brings the locking member 10 into surface contact with the body surface 33 to be locked, thereby positioning the lifted intestinal tract 32 at a position suitable for constructing the artificial anus 34 with respect to the body surface 33.

The operator incises the top portion 32c of the intestinal tract 32 to form a top portion opening 32d through which an opening on an oral side intestinal tract 32X and an opening on an anal side intestinal tract 32Y can be exposed, as shown in FIG. 9.

Then, as shown in FIG. 10, the operator grasps an edge portion defining the top portion opening 32d and reverses the edge portion with reference to an upper end portion of the external cover part 11c to be folded (wound) radially outward. Furthermore, the operator passes a suture thread (not shown) through the passage holes 10b of the locking member 10 shown in FIG. 2 to suture and fix the open end portion 32e of the intestinal tract 32 and the body surface 33 to each other, thereby forming a peripheral edge portion 34a of the artificial anus 34.

Then, the operator passes a suture thread (not shown) through the through-holes 10a of the locking member 10 to suture and fix the locking member 10 and the body surface 33 to each other.

In this manner, the artificial anus 34 is constructed at the open end portion 32e.

In the present embodiment, an example in which a suture thread (not shown) is passed through a thickness of the abdominal wall 30 is shown.

Furthermore, the suture thread may also be used to cause the passage holes 10b in the locking member 10 to be entwined with surrounding parts after the suture thread is passed through the abdominal wall 30, or may further be entwined with only a part around the passage holes 10b in the locking member 10 without passing through the abdominal wall 30.

Thereafter, the pouch 2 configured to store digestive juices, feces, and the like is connected to the locking member 10 of the organ protecting member 1 provided in the periphery of the artificial anus 34. The subject is hospitalized for about two weeks until it is found that there is no suture failure at a site where the lesion portion is excised and sutured. In the meantime, the pouch 2 is exchanged about once every two days.

### <Method of Closing Artificial Anus (Process)>

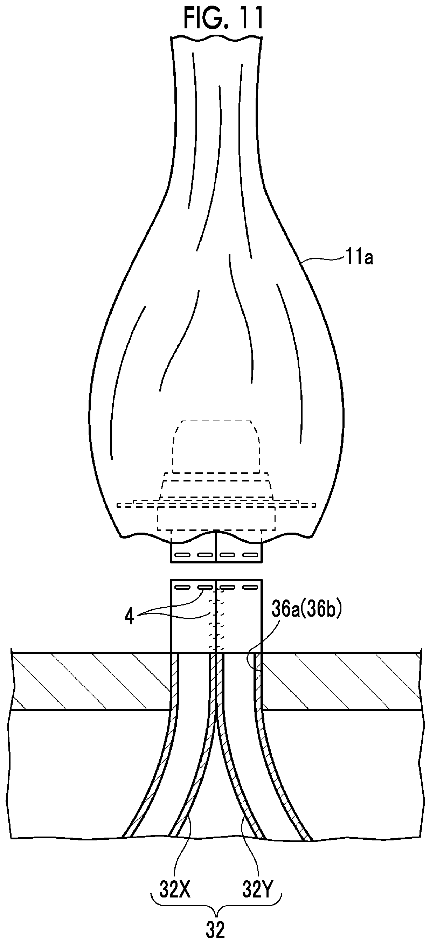

Next, a method of closing an artificial anus will be described mainly with reference to FIG. 11.

FIG. 11 shows an operation of closing the artificial anus 34, shows a state in which the internal cover part 11a is taken out of the body, and is an explanatory view showing an anastomosed portion between the oral side intestinal tract 32X and the anal side intestinal tract 32Y and sutured and cut portions.

The method of closing an artificial anus according to the present embodiment is a method including a closing process of closing the artificial anus 34 constructed by the method of constructing an artificial anus.

In the present embodiment, the operator closes the artificial anus 34 within about 14 days after constructing the artificial anus 34.

In the closing process, specifically, the operator first detaches the pouch 2 from the locking member 10.

Next, the operator cuts the suture thread (not shown) that fixes the locking member 10 and the body surface 33 to each other.

Next, as shown in FIG. 11, the operator takes out the internal cover part 11a inside the abdominal cavity 35 from the body through the opening portion 36a.

Furthermore, the operator turns the internal cover part 11a inside out so as to cover the artificial anus 34 and pulls up the internal cover part 11a to lift the intestinal tract 32. At this time, the operator removes the support string 12 that supports the intestinal tract 32 from below.

Next, the operator makes a small incision in opposing side walls of the oral side intestinal tract 32X and the anal side intestinal tract 32Y in the vicinity of the artificial anus 34 on a side closer to the body surface 33 than the turned internal cover part 11a. Then, the operator passes an automatic anastomosis device (linear stapler) (not shown) through the small incision portion and anastomoses the oral side intestinal tract 32X and the anal side intestinal tract 32Y with staples 4 side by side. In this manner, a new path for feces is formed.

In FIG. 11, the staples 4 provided inside the oral side intestinal tract 32X and the anal side intestinal tract 32Y are indicated by dashed lines.

Next, the operator anastomoses the intestinal tract 32 on an upper side anastomosed side by side (a side away from the body surface 33) immediately below the locking member 10 (on the body surface 33 side) on the side closer to the body surface 33 than the turned internal cover part 11a with the staples 4 using the automatic anastomosis device and cuts the intestinal tract 32.

Last, the operator returns the lifted intestinal tract 32 into the abdominal cavity 35 and closes the opening portion 36a of the abdomen 36.

In the embodiment described above, an example of anastomosis using the automatic anastomosis device is described, but anastomosis may be performed by hand sewing.

### <Second Embodiment>

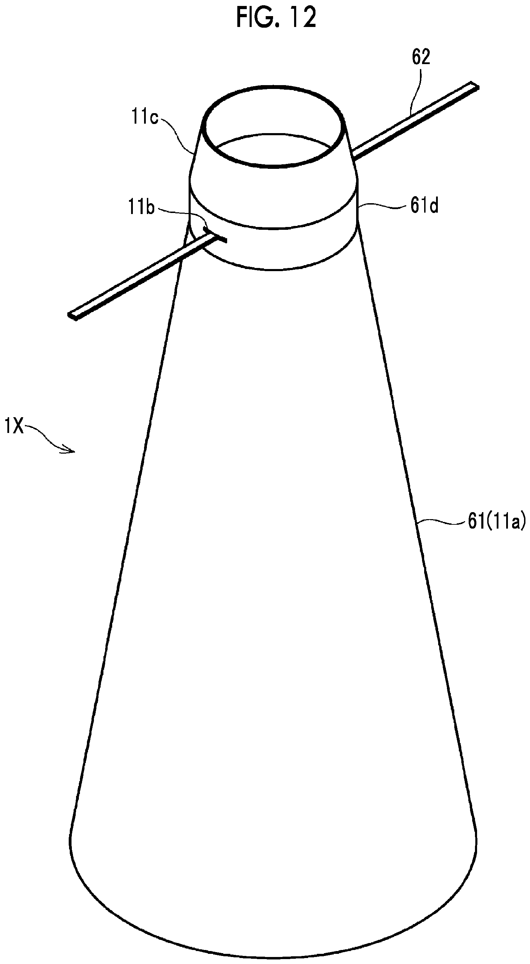

Next, an organ protecting member 1X according to a second embodiment will be described mainly with reference to FIGS. 12 to 14. The organ protecting member 1X differs from the organ protecting member 1 according to the first embodiment in that the organ protecting member 1X does not include the locking member 10, and there are cases where description of the same configuration as those of the first embodiment is omitted.

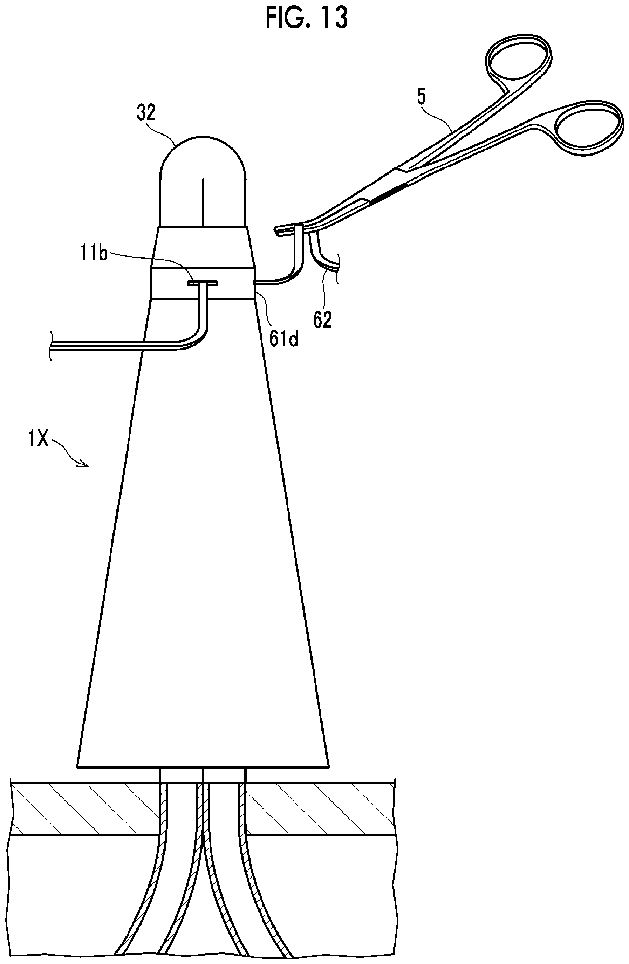

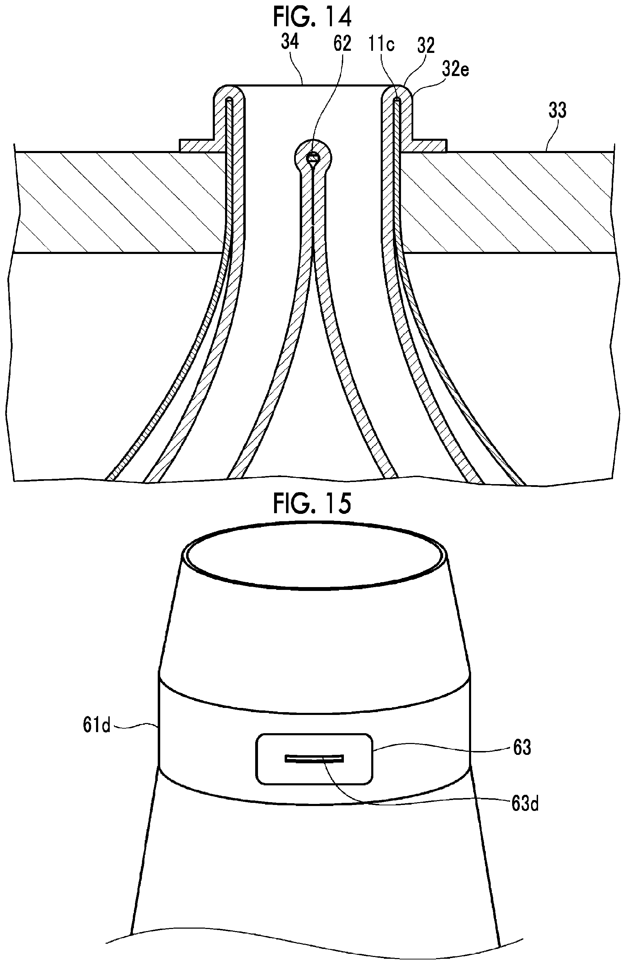

FIG. 12 is a perspective view showing the organ protecting member 1X according to the second embodiment, FIG. 13 is an explanatory view showing an operation of passing a support string 62 according to the second embodiment through the intestinal tract 32 and a cover 61, and FIG. 14 is an explanatory view showing a state in which the intestinal tract 32 is fixed to the body surface 33 (with a suture thread (not shown)).

As shown in FIG. 12, a movement restricting part according to the present embodiment includes the support string 62 that is passed through the slit 11b formed in the cover 61 and is disposed over an inside and an outside of the cover 61.

As shown in FIG. 13, the support string 62 is passed under a portion (top portion 32c) of the intestinal tract 32 lifted to the outside of the body from the body surface 33 side to support the portion of the intestinal tract 32 from below.

Two slits 11b according to the present embodiment are formed at positions shifted by 180 degrees in a circumferential direction of a straight cylindrical part 61d. However, the slit 11b is not limited to this configuration, and only one slit 11b may be formed in the straight cylindrical part 61d.

In this case, for example, one end of the support string 62 may be fixed to a portion of the cover 61 (for example, an inner surface of the straight cylindrical part 61d) and the other end of the support string 62 may be passed through the slit 11b.

According to the above configuration, movement of the intestinal tract 32 can be restricted by passing the support string 62 through the slit 11b of the cover 61, even if the organ protecting member 1X does not include the locking member 10 according to the first embodiment.

In particular, the support string 62 according to the present embodiment is formed to have a wide rectangular cross section. Therefore, the support string 62 can be adapted to the slit 11b elongated in the circumferential direction, and formation of a gap therebetween can be suppressed. In addition, in a case where the intestinal tract 32 tries to return to the inside of the body, during pressing in a direction perpendicular to the support string 62, a contact area of the support string 62 can be larger than that of a support string having a circular cross section, and a load can be distributed.

In addition, both ends of the support string 62 may be tied above the artificial anus 34, or may be disposed radially outside the artificial anus 34 and pinched with a body surface fixing portion (not shown) provided in the pouch 2 to be fixed.

In the organ protecting member 1X, a maximum diameter portion of a portion (the external cover part 11c and the straight cylindrical part 61d) exposed to the body surface 33 is a portion of the cover 61 in which the slit 11b is formed (straight cylindrical part 61d).

Unlike the organ protecting member 1 according to the first embodiment, the organ protecting member 1X is not provided with the locking member 10, so that the organ protecting member 1X can suppress bulkiness around the artificial anus 34.

In addition, after forming the external cover part 11c to be long in an axial direction and passing the support string 62 through the inner peripheral side 32a (mesentery) of the top portion 32c of the intestinal tract 32 having a U shape, the external cover part 11c may be cut with scissors to adjust a height thereof so as to match a height of an artificial anus construction portion.

Since the organ protecting member 1X according to the present embodiment does not include the locking member 10, as shown in FIG. 14, a suture thread (not shown) is directly sutured to a folded portion of the open end portion 32e and the body surface 33 without being passed through the passage holes 10b of the locking member 10.

At this time, in the first embodiment, it is described that the intestinal tract 32 is folded together with the external cover part 11c. Here, in the present embodiment, it is possible to determine whether or not to fold the external cover part 11c as needed. As shown in FIG. 14, only the open end portion 32e of the intestinal tract 32 may be folded without folding the external cover part 11c.

Then, the pouch 2 is directly attached to the body surface 33 instead of the locking member 10 and is attached so as to cover the support string 62.

### (First Modification Example)

Since the support string 62 is passed through the slit 11b, it is necessary to prevent a backflow of feces in the pouch 2 from the artificial anus 34 into the body along an inner surface of the cover 61 through the slit 11b. Of a first modification example and a second modification example from this viewpoint, the first modification example will be described first mainly with reference to FIG. 15.

FIG. 15 is an explanatory view describing a reinforcing seal 63 according to the first modification example.

In the present example, a reinforcing member (reinforcing seal 63) is provided around the slit 11b.

The reinforcing seal 63 is suitably formed of a material having higher rigidity than the cover 61.

However, the reinforcing seal 63 is not limited to such a configuration, and it is sufficient if the rigidity around the slit 1 1b can be increased. That is, although the reinforcing seal 63 may be made of the same material as the cover 61, the rigidity around the slit 11b may be increased by increasing a thickness around the slit 11b.

In addition, in the reinforcing seal 63, a circumferentially elongated slit 63d having a shape along the slit 11b is formed.

By attaching the reinforcing seal 63 to the cover 61 so that the slit 63d of the reinforcing seal 63 overlaps the slit 11b of the cover 61, deformation of a peripheral edge of the slit 11b is suppressed (to make the slit 11b difficult to open), and generation of a gap between the peripheral edge of the slit 11b and the support string 62 can be prevented.

For example, the cover 61 may have a marker and the reinforcing member (reinforcing seal 63) may have a marker different from the marker of the cover 61.

A "marker" is one that can be visually or tactilely identified by a user, such as color, material, or surface finish.

According to such a configuration, a position of the slit 11b, which is an opening and is not easily recognized as an outer shape, is made conspicuous by the reinforcing seal 63 and thus can be easily recognized by the user.

### (Second Modification Example)

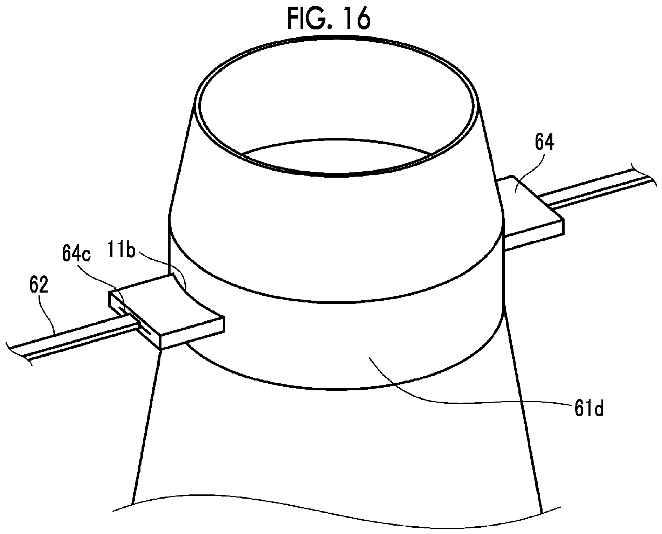

The second modification example will be described mainly with reference to FIG. 16. FIG. 16 is an explanatory view showing a seat valve 64 according to the second modification example.

In the present example, an interposed material (seat valve 64) is provided between an inner periphery of the slit 11b and the support string 62. The seat valve 64 narrows the gap between the support string 62 passed through the slit 11b and the slit 11b.

The seat valve 64 is made of, for example, silicone rubber, is attached to an inner edge surface of the slit 11b of the cover 61, and extends in a direction perpendicular to the slit 11b (a direction perpendicular to an axial direction of the cover 61).

Specifically, the interposed material (seat valve 64) has a strip shape (thin film shape with a slit 64c) and is formed to be wider than the support string 62.

By passing the support string 62 through the slit 64c of the seat valve 64, the support string 62 is supported in a state of being in close contact with the inner edge surface of the slit 64c.

The seat valve 64 may be configured by combining an upper seat valve (not shown) and a lower seat valve (not shown) so as to overlap each other in a plate thickness direction. In this case, the support string 62 is supported from above and below by passing the support string 62 between the upper seat valve and the lower seat valve.

Since the seat valve 64 is formed in a strip shape with a large width, when a pressure of feces is applied from an inside of the intestinal tract 32, a load in a thickness direction of the seat valve 64 is applied over a wide range. Therefore, the seat valve 64 can be brought into close contact with the support string 62, and can prevent feces from passing through the slit 11b.

Moreover, the gap between the slit 11b and the support string 62 can be suitably buried while suppressing bulkiness of the seat valve 64 in the thickness direction.

Although it is described that the seat valve 64 is attached to the inner edge surface of the slit 11b of the cover 61, the seat valve 64 is not limited to such a configuration, may be provided on a portion of an outer periphery of the support string 62 (a portion passed through the slit 11b).

In particular, in a case where the seat valve 64 is made of an elastic material, the inner edge surface of the slit 11b and an outer peripheral surface of the support string 62 are easily brought into close contact with each other, which is preferable.

In addition, after passing the support string 62 through the slit 11b, the seat valve 64 may be inserted therebetween like a wedge.

### (Third Modification Example)

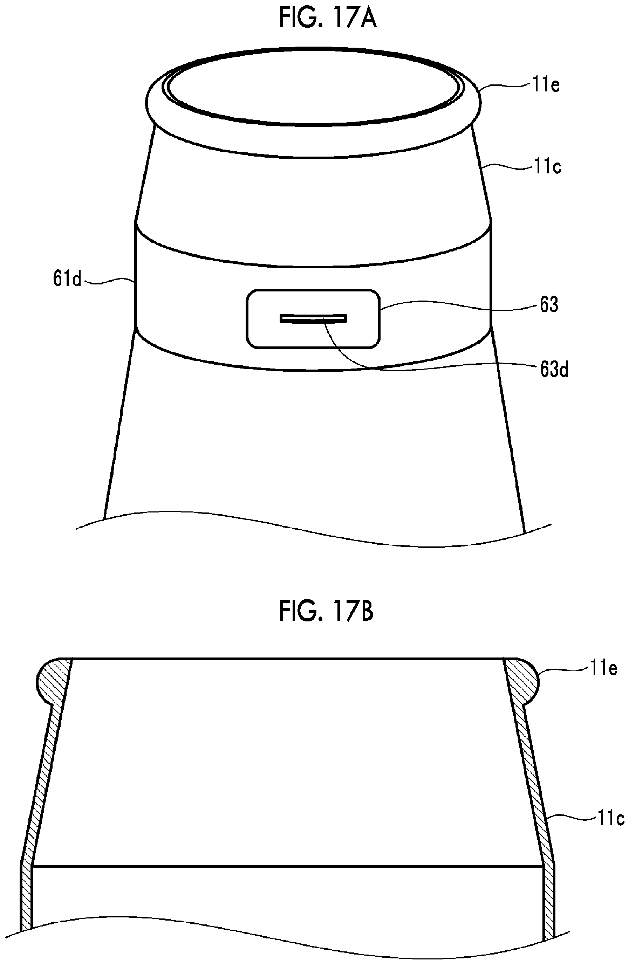

A suture reinforcing part 11e of the external cover part 11c according to a third modification example will be described mainly with reference to FIGS. 17A and 17B. FIG. 17A is a perspective view describing the suture reinforcing part 11e of the external cover part 11c according to the third modification example. FIG. 17B is a longitudinal cross-sectional view of FIG. 17A.

In the external cover part 11c according to the present example, the suture reinforcing part 11e for reinforcing a sutured portion to which a portion of the intestinal tract 32 is sutured is formed. The suture reinforcing part 11e is formed thicker than surroundings thereof.

More specifically, the suture reinforcing part 11e according to the present example is a thick protruding portion that is formed around an entire circumference of an upper end portion of an outer peripheral surface of the external cover part 11c, and is swollen radially outward on the external cover part 11c.

In other words, "reinforcing the sutured portion" described above means that by the suture reinforcing part 11e formed to be thick in the vicinity of the sutured portion (within a range in which tension of a suture thread (not shown) forming the sutured portion is applied), stress applied from the suture thread to the external cover part 11c is distributed and thus release of the suturing is limited.

According to the above configuration, since the external cover part 11c is provided with the suture reinforcing part 11e that is thicker than the surroundings thereof, strength of the sutured portion where the external cover part 11c and the intestinal tract 32 are sutured to each other with the suture thread (not shown) can be increased.

Although it is described that the suture reinforcing part 11e is formed around the entire circumference of the upper end portion of the external cover part 11c, but the suture reinforcing part 11e is not limited to such as configuration as long as the sutured portion (not shown) can be reinforced. For example, the suture reinforcing part 11e may be provided not at the upper end portion of the external cover part 11c but at a central portion or the like, and may also be formed locally (for example, in an arc shape) in the circumferential direction.

In a case where the suture reinforcing part 11e is formed so as to protrude from an outer surface of the external cover part 11c, the suture reinforcing part 11e does not narrow an opening, which is preferable. However, the suture reinforcing part 11e is not limited to such a configuration, and the suture reinforcing part 11e may be configured to be formed on an inner surface of the external cover part 11c so as to be disposed at a position exposed to the outside without narrowing the opening by folding the external cover part 11c.

In addition, the suture reinforcing part 11e may be made of the same material as that of the external cover part 11c, but may be made of a different material. The different material may be a material different from that of the external cover part 11c (for example, a hard resin member such as polycarbonate or hard polyvinyl chloride).

In addition, the suture reinforcing part 11e can serve not only as a reinforcement of the sutured portion but also as a guide for a sutured position (a sutured position from an end portion of the external cover part 11c). In particular, even in a state of being covered with the intestinal tract 32, the sutured position of the external cover part 11c can be specified by the suture reinforcing part 11e, which is preferable.

As a matter of course, the suture reinforcing part 11e according to the third modification example can be applied to the external cover part 11c according to the first embodiment.

The present invention includes the following aspects.
(1) An organ protecting member used when an opening portion is formed in an abdominal wall and an intestinal tract is exposed to a body surface from the opening portion to temporarily construct an artificial anus, including:
   a movement restricting part configured to restrict movement of a portion of the intestinal tract on which the artificial anus is constructed toward an abdominal cavity; and
   a cover disposed between the intestinal tract and an inner wall surface of the opening portion.
(2) The organ protecting member according to (1), in which the cover is formed in a shape that flares outward from an external side toward an internal side.
(3) The organ protecting member according to (1) or (2), in which the movement restricting part includes a support string that is passed through a slit formed in the cover and is disposed over an inside and an outside of the cover.
(4) The organ protecting member according to (3), in which a maximum diameter portion of a portion exposed to the body surface is a portion of the cover in which the slit is formed.
(5) The organ protecting member according to (4), in which a reinforcing member is provided around the slit.
(6) The organ protecting member according to (5), in which the cover has a marker, and the reinforcing member has another marker different from the marker.
(7) The organ protecting member according to any one of (4) to (6), in which an interposed material is provided between an inner periphery of the slit and the support string.
(8) The organ protecting member according to (7), in which the interposed material is formed in a strip shape.
(9) The organ protecting member according to (1) or (2), in which the movement restricting part includes a locking member that is connected to the cover and comes into surface contact with and locks the body surface.
(10) The organ protecting member according to (9),
   in which the locking member is formed in a plate piece shape, and
   in the locking member, a through-hole through which a suture thread used to suture and fix the locking member to the body surface is passed is formed in a thickness direction.
(11) The organ protecting member according to (9) or (10),
   in which the locking member is formed in a plate piece shape, and
   in the locking member, a passage hole through which a suture thread used to suture and fix a portion of the intestinal tract forming a periphery of the artificial anus to the body surface is passed is formed in a thickness direction.
(12) The organ protecting member according to any one of (9) to (11),
   in which the cover includes an internal cover part disposed on an internal side and an external cover part disposed on an external side with respect to a portion of the cover connected to the locking member, and
   the external cover part is fixed to the portion of the intestinal tract on which the artificial anus is constructed.
(13) The organ protecting member according to any one of (9) to (12), in which the movement restricting part includes a support string that is passed under a portion of the intestinal tract lifted to an outside of a body and supports the portion of the intestinal tract from below.
(14) The organ protecting member according to (13),
   in which the locking member includes an extending part extending toward an inside of the body, and
   a hole through which the support string is passed is formed in the extending part and the cover.
(15) The organ protecting member according to any one of (1) to (14),
   in which the cover includes an internal cover part disposed on an internal side and an external cover part disposed on an external side,
   a suture reinforcing part configured to reinforce a sutured portion to which a portion of the intestinal tract is sutured is formed in the external cover part, and
   the suture reinforcing part is formed thicker than surroundings of the suture reinforcing part.
(16) The organ protecting member according to any one of (1) to (15),
   in which the cover includes an internal cover part disposed on an internal side, and
   pleats extending in a longitudinal direction are formed in the internal cover part.
(17) A method of constructing an artificial anus in which an opening portion is provided in an abdominal wall and an intestinal tract is lifted from the opening portion to construct an artificial anus, including:
   preparing an organ protecting member including a movement restricting part configured to restrict movement of a portion of the intestinal tract on which the artificial anus is constructed toward an abdominal cavity, and a cover disposed between the intestinal tract and an inner wall surface of the opening portion;
   an incision process of forming the opening portion in the abdominal wall;
   a construction process of exposing the intestinal tract from the opening portion to a body surface and constructing the artificial anus by disposing at least a portion of the cover between the intestinal tract and the inner wall surface of the opening portion.
(18) The method of constructing an artificial anus according to (17),
   in which the movement restricting part has a locking member connected to the cover and locked to the body surface,
   the cover is connected to the intestinal tract, and
   in the construction process, a position of the lifted intestinal tract is maintained by bringing the locking member into surface contact with the body surface to be locked.
(19) The method of constructing an artificial anus according to (18),
   in which the locking member is formed in a plate piece shape,
   in the locking member, a through-hole is formed in a plate thickness direction, and
      in the construction process, a suture thread is passed through the through-hole to suture and fix the locking member to the body surface.
(20) The method of constructing an artificial anus according to (18) or (19),
   in which the locking member is formed in a plate piece shape,
   in the locking member, a passage hole is formed in a plate thickness direction, and
      in the construction process, a suture thread is passed through the passage hole to suture and fix a portion of the intestinal tract forming a peripheral edge portion of the artificial anus to the body surface.
(21) The method of constructing an artificial anus according to any one of (17) to (19), in which the cover includes an internal cover part disposed on an internal side and an external cover part disposed on an external side with respect to a portion of the cover connected to the locking member, and
   in the construction process, the external cover part is fixed to a portion of the intestinal tract on which the artificial anus is constructed.
(22) The method of constructing an artificial anus according to (21), in which, in the construction process, the artificial anus is constructed by fixing the external cover part is fixed to the intestinal tract lifted to an outside of a body, forming an opening portion by incising a top portion of the intestinal tract, and reversing the intestinal tract from the opening portion of the intestinal tract.
(23) The method of constructing an artificial anus according to any one of (18) to (22), in which the movement restricting part has a support string that supports the lifted intestinal tract, and
   in the construction process, the support string is passed through the intestinal tract and the cover so as to be passed under the lifted intestinal tract and support the lifted intestinal tract from below.
(24) A method of closing an artificial anus including a closing process of closing the artificial anus constructed by the method of constructing an artificial anus according to any one of (18) to (23),
   in which the cover includes an internal cover part disposed on an internal side with respect to a portion of the cover connected to the locking member, and
   in the closing process, the internal cover part is taken out of a body through the opening portion, the internal cover part is turned inside out so as to cover the artificial anus, the internal cover part is pulled up to lift the intestinal tract, and the intestinal tract is anastomosed.
(25) The method of closing an artificial anus according to (24), in which, in the closing process, the intestinal tract is anastomosed on a side closer to a body surface than the turned internal cover part.

### Reference Signs List

1, 1X: organ protecting meber
10: locking member (movement restricting part)
10a: through-hole
10b: passage hole
10c: extending part
10d: slit (hole)
10e: body surface plate
10f: body surface plate upper portion
10g: body surface plate lower portion
11: cover
11a: internal cover part
11b: slit (hole)
11c: external cover part (movement restricting part)
11d: straight cylindrical part
11e: suture reinforcing part
12: support string (movement restricting part)
12X: string threader
15: suture thread
2: pouch
3: subject
30: abdominal wall
32: intestinal tract (small intestine, ileum)
32X: oral side intestinal tract
32Y: anal side intestinal tract
32a: inner peripheral side
32b: outer peripheral side
32c: top portion
32d: top portion opening
32e: open end portion
33: body surface
34: artificial anus
34a: peripheral edge portion
35: abdominal cavity
36: abdomen
36a: opening portion
36b: inner wall surface
4: staple
5: intestinal forceps
61: cover
61d: straight cylindrical part
62: support string (movement restricting part)
63: reinforcing seal (reinforcing member)
63d: slit
64: seat valve (interposed material)
64c: slit

## Claims

1. An organ protecting member used when an opening portion is formed in an abdominal wall and an intestinal tract is exposed to a body surface from the opening portion to temporarily construct an artificial anus, comprising:
a movement restricting part configured to restrict movement of a portion of the intestinal tract on which the artificial anus is constructed toward an abdominal cavity; and
a cover disposed between the intestinal tract and an inner wall surface of the opening portion.

2. The organ protecting member according to claim 1,
wherein the cover is formed in a shape that flares outward from an external side toward an internal side.

3. The organ protecting member according to claim 1 or 2,
wherein the movement restricting part comprises a support string that is passed through a slit formed in the cover and is disposed over an inside and an outside of the cover.

4. The organ protecting member according to claim 3,
wherein, in the organ protecting member, a maximum diameter portion of a portion exposed to the body surface is a portion of the cover in which the slit is formed.

5. The organ protecting member according to claim 4,
wherein a reinforcing member is provided around the slit.

6. The organ protecting member according to claim 5,
wherein the cover has a marker, and
the reinforcing member has another marker different from the marker.

7. The organ protecting member according to any one of claims 4 to 6,
wherein an interposed material is provided between an inner periphery of the slit and the support string.

8. The organ protecting member according to claim 7,
wherein the interposed material is formed in a strip shape.

9. The organ protecting member according to claim 1 or 2,
wherein the movement restricting part comprises a locking member that is connected to the cover and comes into surface contact with and locks the body surface.

10. The organ protecting member according to claim 9,
wherein the locking member is formed in a plate piece shape, and
in the locking member, a through-hole through which a suture thread used to suture and fix the locking member to the body surface is passed is formed in a thickness direction.

11. The organ protecting member according to claim 9 or 10,
wherein the locking member is formed in a plate piece shape, and
in the locking member, a passage hole through which a suture thread used to suture and fix a portion of the intestinal tract forming a periphery of the artificial anus to the body surface is passed is formed in a thickness direction.

12. The organ protecting member according to any one of claims 9 to 11,
wherein the cover comprises an internal cover part disposed on an internal side and an external cover part disposed on an external side with respect to a portion of the cover connected to the locking member, and
the external cover part is fixed to the portion of the intestinal tract on which the artificial anus is constructed.

13. The organ protecting member according to any one of claims 9 to 12,
wherein the movement restricting part comprises a support string that is passed under a portion of the intestinal tract lifted to an outside of a body and supports the portion of the intestinal tract from below.

14. The organ protecting member according to claim 13,
wherein the locking member comprises an extending part extending toward an inside of the body, and
a hole through which the support string is passed is formed in the extending part and the cover.

15. The organ protecting member according to any one of claims 1 to 14,
wherein the cover comprises an internal cover part disposed on an internal side and an external cover part disposed on an external side,
a suture reinforcing part configured to reinforce a sutured portion to which a portion of the intestinal tract is sutured is formed in the external cover part, and
the suture reinforcing part is formed thicker than surroundings of the suture reinforcing part.

16. The organ protecting member according to any one of claims 1 to 15, wherein the cover comprises an internal cover part disposed on an internal side, and pleats extending in a longitudinal direction are formed in the internal cover part.
